# DEMANDE DE BREVET EUROPEEN

(11) **EP 1 616 853 A1**
(43) Date de publication de la demande: **18.01.2006**
(21) Numéro de dépôt: 05291347.2
(22) Date de dépôt: 23.06.2005
(51) Int. Cl.: C07C 67/56, C07C 67/03, C07C 69/52, C07C 69/24, C11C 3/10

(54) **Procédé de production d'esters alkyliques d'acides gras et de glycérol de haute pureté**

(30) Priorité: 12.07.2004 FR 0407777
(71) Demandeur: Institut Français du Pétrole, 92852 Rueil Malmaison Cédex (FR)
(72) Inventeur: Bournay, Laurent, 69440 Chaussan (FR); Baudot, Arnaud, 69003 Lyon (FR)

(57) **Abrégé**

Dans un procédé de production d'esters alkyliques d'acide gras et de glycérol comprenant
- au moins une étape réactionnelle dans laquelle on met en contact une charge comportant une huile végétale et/ou animale et un alcool, en présence d'un catalyseur hétérogène, de manière à obtenir un effluent comportant au moins des esters alkyliques, du glycérol et de l'alcool,
- et au moins une étape de séparation lors de laquelle on réalise une séparation d'au moins une portion de l'effluent de manière à séparer un effluent riche en alcool et un effluent riche en esters alkyliques,
au moins une étape de séparation consiste en une séparation membranaire utilisant au moins une membrane perméable à l'alcool.

## Description

### Domaine de l'invention

L'invention concerne le domaine des procédés de fabrication d'esters alkyliques d'acide gras et de glycérol par mise en contact d'une huile végétale et/ou animale et d'un alcool aliphatique, en présence d'un catalyseur hétérogène.

### Art antérieur

L'utilisation des esters méthyliques d'huiles végétales, en abrégé EMVH, comme carburants de substitution au gazole est promise à un large développement dans les deux prochaines décennies. En effet, la présence dans ce produit de monoglycérides sous forme de traces (0,8 % masse maximum selon la norme en vigueur), permet de compenser avantageusement la perte de pouvoir lubrifiant due notamment à la réduction de la teneur en soufre dans le gazole. La teneur en soufre sera réglementée en Europe à 50 ppm masse en 2005 et 10 ppm masse en 2008.

Par ailleurs, la Commission européenne a adopté un plan d'action et deux propositions de directive en vue d'encourager l'utilisation des carburants de substitution dans le secteur des transports, en commençant par des mesures réglementaires et fiscales destinées à promouvoir les biocarburants. Le plan d'action définit une stratégie permettant de remplacer, d'ici à 2020, 20 % du carburant diesel et de l'essence par des carburants de substitution dans le secteur des transports routiers. Une des directives proposées prévoit que les biocarburants représentent une proportion minimale de l'ensemble des carburants vendus à partir de 2005. Cette proportion serait de 2 % initialement et atteindrait 5,75 % en 2010.

La production d'esters méthyliques d'huiles végétales ou Biodiesel, essentiellement à partir d'huile de Colza, dépasse les 300 000 t/an en France. De plus, il existe d'autres applications possibles de ces produits telles que les solvants écologiques, et des composés de base pour la fabrication de sulfonates d'alcool gras, d'amides, de dimères d'esters, etc.

La demande de brevet européen EP-A-1 352 893 décrit un procédé comprenant la mise en oeuvre d'une réaction d'estérification d'une huile végétale ou d'une huile d'origine animale au moyen d'un monoalcool aliphatique utilisé en excès, avec séparation et recyclage de l'excès de monoalcool aliphatique, pour donner du glycérol et de l'ester alkylique, en présence d'un catalyseur hétérogène.

Du fait de la nature très polaire du méthanol, son enthalpie de vaporisation est relativement élevée, environ 1200 kJ/kg à température ambiante et 610 kJ/kg à 200°C, et par conséquent son extraction du milieu réactionnel par vaporisation est très consommatrice d'énergie. De plu, pour être recyclé dans le milieu réactionnel, ce méthanol doit être condensé pour être ensuite comprimé par une pompe sous forme liquide.

### Résumé de l'invention

Un objet de l'invention est de fournir un procédé beaucoup plus économique, permettant, en particulier, de pallier, au moins partiellement, la consommation d'énergie rédhibitoire d'une étape de séparation par vaporisation.

Il a été trouvé un procédé de fabrication d'esters alkyliques d'acide gras et de glycérol mettant en oeuvre une ou plusieurs réactions de transestérification entre une huilé végétale et un alcool, dans lequel on sépare un effluent riche en alcool d'un effluent riche en esters alkyliques par une séparation de type membranaire.

Les réactions de transestérification mises en oeuvre dans la présente invention comprennent généralement :
- la réaction de l'huile, triglycéride, avec une molécule d'alcool pour donner une molécule d'ester et un diglycéride ;
- la réaction du diglycéride avec une molécule d'alcool pour donner une molécule d'ester et un monoglycéride, ; et
- la réaction du monoglycéride avec une molécule d'alcool pour donner une molécule d'ester et une molécule de glycérol.

Ces réactions sont généralement équilibrées et peuvent se dérouler parallèlement pendant l'ensemble de la mise en oeuvre du procédé de l'invention.

Les membranes utilisées dans l'étape de séparation membranaire du procédé de l'invention sont perméables à l'alcool, ce dernier étant généralement recyclé dans le milieu réactionnel. Ces membranes peuvent être, en outre, perméables également au glycérol.

Dans un mode préféré de l'invention, le procédé comporte deux étapes réactionnelles, chacune de ces étapes réactionnelles étant suivie par au moins une étape de séparation membranaire.

### Brève description des figures

La Figure 1 représente, de manière non limitative, un mode de réalisation simplifié du procédé de l'invention comportant une étape réactionnelle et une étape de filtration membranaire.
La Figure 2 représente, de manière non limitative, un mode de réalisation comportant, en outre, un recyclage de l'alcool dans l'étape réactionnelle.
La Figure 3 représente, de manière non limitative, un mode de réalisation comportant, en outre, une étape supplémentaire de purification de l'alcool avant le recyclage de ce dernier.
La Figure 4 représente, de manière non limitative, un mode de réalisation comportant, en outre, une étape de séparation de l'alcool issu du retentat.
La Figure 5 représente, de manière non limitative, un enchaînement pour la mise en oeuvre industrielle du procédé de l'invention.
Les Figures 6 et 7 sont données pour illustrer respectivement un premier exemple non conforme à l'invention et un deuxième exemple conforme à l'invention.

### Description détaillée de l'invention

La présente invention fournit donc un procédé de fabrication d'esters alkyliques d'acide gras et de glycérol qui comprend :
- au moins une étape réactionnelle dans laquelle on met en contact une charge comportant une huile végétale et/ou animale et un alcool, en présence d'un catalyseur hétérogène, de manière à obtenir un effluent comportant au moins des esters alkyliques, du glycérol et de l'alcool,
- et au moins une étape de séparation lors de laquelle on réalise une séparation d'au moins une portion de l'effluent de manière à séparer un effluent riche en alcool et un effluent riche en esters alkyliques, ledit procédé étant caractérisé en ce qu'au moins une étape de séparation comporte une séparation membranaire à l'aide d'au moins une membrane perméable à l'alcool.

Par ester alkylique d'acide gras, on entend un ester issu de l'estérification d'un alcool aliphatique, en général saturé, sur un acide carboxylique provenant d'un glycéride d'origine végétale ou animale.

Par glycérol, on entend la molécule du tri-alcool ayant trois atomes de carbone dont la formule chimique est C₃H₈O₃. Le glycérol selon l'invention peut également se trouver sous forme de glycérine. La glycérine peut être définie comme étant un mélange comportant au moins 50 % en poids de glycérol ainsi que de l'eau, méthanol, des sels, des matières organiques sans glycérol.

Les huiles végétales et/ou animales qui peuvent être utilisées dans le procédé de l'invention peuvent être tout huile connu de l'homme du métier, par exemple de l'huile de colza, de palme, de tournesol, de soja, de copra, de ricin, ainsi que des huiles d'origine animales comme le suif.

L'alcool mis en oeuvre dans le procédé de l'invention est généralement un alcool aliphatique. L'alcool du procédé de l'invention est le plus souvent un monoalcool aliphatique. De préférence, l'alcool est essentiellement constitué par du méthanol et/ou de l'éthanol.

L'amélioration du procédé de l'invention porte essentiellement sur le fait qu'il met en oeuvre une étape de séparation comportant une séparation membranaire à l'aide d'au moins une membrane perméable à l'alcool.

Parmi les technologies de séparation membranaire basées sur un transport sélectif d'alcool, tel que du méthanol, à travers un film grâce à un mécanisme de sorption-diffusion, celles mettant en oeuvre une perméation de vapeur ou une pervaporation peuvent être envisagées.

Les technologies basées sur la perméation de vapeur sont généralement des séparations menées sur une charge en phase gazeuse basées sur la perméation sélective d'une fraction des composés de ladite charge, appelée le perméat, à travers un film dense. Les phénomènes mis en jeu sont généralement une sorption et une diffusion induite par une différence de potentiel chimique.

Les technologies basées sur la pervaporation sont généralement des séparations menées sur une charge en phase liquide. La pervaporation est basée sur une perméation sélective d'une fraction des composés de ladite charge, appelée le perméat, à travers une membrane constituée essentiellement d'un film dense, ladite perméation étant associée à une vaporisation de ce perméat en aval de la membrane. La vaporisation est généralement réalisée soit par un balayage du compartiment en aval de la membrane à l'aide d'un gaz inerte, soit par une mise sous vide partiel de ce compartiment en aval de la membrane. De la même façon que pour la perméation de vapeur, les phénomènes mis en jeu sont généralement une sorption et une diffusion induite par une différence de potentiel chimique.

Le gain en terme de consommation énergétique de ces deux technologies est toutefois limité car elles nécessitent soit une vaporisation totale de la charge dans le cas de la perméation de vapeur, soit une vaporisation d'une grande quantité de l'alcool sous forme de perméat dans le cas de la pervaporation.

On préfère généralement utiliser des techniques de séparation membranaire mettant à profit le gisement de pression mécanique contenu dans l'effluent en sortie du réacteur, et ceci dans le but de réaliser une opération de filtration hyperbare permettant une perméation sélective du méthanol seul, ou bien du méthanol et du glycérol, à travers une membrane.

De préférence, le procédé de l'invention comporte au moins une séparation membranaire par nano-filtration et/ou par osmose inverse.

Ces techniques de filtration membranaire sont particulièrement bien adaptées à la séparation des composés ayant une petite taille des composés plus encombrants, par exemple la séparation du méthanol d'un mélange de glycérol, d'esters et de glycérides, ou bien la séparation d'un mélange de méthanol et de glycérol d'un mélange d'esters et de glycérides.

Les membranes de nano-filtration présentent généralement une taille effective de pores allant de 5 à 50 angströms, alors que les membranes d'osmose inverse présentent une taille effective de pores inférieure à 10 angströms.

La nano-filtration et l'osmose inverse sont généralement basées sur un mécanisme de ségrégation stérique associé à des effets d'affinité chimique. Le flux des espèces traversant la couche sélective de la membrane est principalement de nature convective et ce flux peut être induit par une différence de pression mécanique entre les faces amont et.aval de la membrane.

Les composés traversant la membrane (désignés par "le perméat") sont généralement récupérés dans un compartiment à basse pression, en aval de la membrane, alors que le "rétentat", c'est-à-dire l'ensemble des composés ne traversant pas la membrane, est généralement maintenu dans un compartiment à haute pression en amont de la membrane.

Dans le procédé de l'invention, la filtration membranaire présente de nombreux avantages, tels que :
- le maintien de l'effluent du réacteur sous pression au cours de l'extraction de l'alcool et
- la réduction des coûts qui étaient liés à la vaporisation et à la condensation de l'alcool, ce dernier étant, dans le cas de l'invention, directement récupéré en phase liquide en aval de la membrane.

Ainsi, si une deuxième étape réactionnelle est nécessaire pour optimiser la conversion en huile, une réduction des coûts est induite par le maintien sous pression de l'effluent riche en esters alkyliques qui est retenue en amont de la membrane de l'étape de séparation membranaire entre la première et la deuxième étape réactionnelle.

La pression en amont de la membrane peut être maintenue de 1 à 12 MPa, de manière préférée de 2 à 8 MPa, de manière plus préférée de 4 à 6 MPa. La pression en aval peut aller, quant à elle, de 0,1 et 3,5 MPa, de manière préférée de 0,1 à 2 MPa, de manière plus préférée de 0,1 à 0,5 MPa.

Selon un mode de réalisation du procédé de l'invention, la (ou les) membrane(s) mise(s) en oeuvre dans l'étape de séparation est (sont), au moins partiellement, perméable(s) au glycérol.

De préférence, au moins une membrane comporte au moins un film à base de polymère. De nombreux modèles commerciaux sont disponibles et sont susceptibles de convenir pour l'application envisagée du fait de leur grande résistance chimique en milieu solvant.

Le Tableau 1 ci-dessous présente une liste non exhaustive des types de membranes commerciales pouvant convenir à la séparation membranaire de l'invention.

**Tableau 1**

| Fabricant | Modèle commercial | Nature de la couche sélective | Technologie |
|---|---|---|---|
| Celgard¹ | N30F | Polyéthersulfone | NF |
| | NF-PES-10 | Polyéthersulfone | NF |
| Koch Membrane Systems, Inc.² | MPF 44 | Silicone | NF |
| | MPF 50 | Silicone | NF |
| Toray³ | UTC 60 | PA | NF |
| | UTC 70 | PA | NF |
| Nitto⁴ | NTR 729HF | PVA / PA | OI basse pression |
| | LF 10 | PVA / PA | OI basse pression |
| | ES 10C | PVA / PA | OI basse pression |
| MET⁵ (Grace Davison) | STARMEM 120 | Polyimide | NF |
| | STARMEM 122 | Polyimide | NF |
| | STARMEM 228 | Polyimide | NF |

| | | | |
|---|---|---|---|
| NF = nano-filtration, OI = osmose inverse, PVA = alcool polyvinylique, | | | |
| PA = polyamide | | | |
| ¹ Celgard Inc. 13800 South Lakes Drive Charlotte, NC 28273, USA | | | |
| ² Koch Membrane Systems, Inc. 850 Main Street Wilmington, MA USA | | | |
| ³ Toray Membrane Products Dept. Head Office Toray Building, 8-1, Mihama 1- chome, Urayasu, Chiba 279-8555, Japon | | | |
| ⁴ Nitto Europe NV, Eikelaarstraat 22, Ind. Park Zuid, Zone 12A, 3600 Genk, Belgique | | | |
| ⁵ Membrane Extraction Technol. Ltd, Dpt Chem. Eng., Imperial College, Londres SW7 2BY, Grande Bretagne | | | |

Parmi ces membranes à base de polymère, on préfère utiliser, par ordre de préférence, les suivantes :
- STARMEM 120 de MET (Grace Davison)
- NF-PES-10 de Celgard et
- UTC 60 de Toray.

Dans le cas d'une membrane en polymère, il est recommandé de réaliser la séparation membranaire :
- à une température généralement supérieure à la température ambiante, et inférieure à 100°C, de préférence inférieure à 80°C, de manière plus préférée à une température allant de 40 à 65°C,
- à une pression de rétentat inférieure à 15 MPa, de préférence inférieure à 7 MPa, et
- à une pression de perméat comprise entre celle du rétentat et la pression d'ébullition du perméat à la température du module, c'est-à-dire souvent inférieure à 0,5 MPa.

De manière préférée, la membrane comporte un film à base de polymère ayant subi un traitement physique (par cuisson ou par traitement aux rayons UV par exemple) ou chimique de réticulation. Le mécanisme de réticulation consiste à créer des ponts chimiques entre les chaînes de polymère constituant la couche sélective de la membrane. Ce traitement de réticulation conduit à deux améliorations technologiques sur les membranes à base de polymère. En effet, la réticulation réduit significativement les phénomènes de gonflement de la matrice polymère constitutive de la couche sélective de la membrane induit par les composés organiques traversant ladite membrane. D'une part, l'effet de tamisage du film polymère se trouve donc renforcé, ce qui se traduit par une augmentation de la sélectivité de la membrane ayant subi le traitement de réticulation. D'autre part, la réduction du gonflement a pour conséquence une meilleure résistance chimique de la membrane et donc une augmentation de sa durée de vie.

De préférence, le procédé de l'invention met en oeuvre au moins une membrane inorganique. Bien qu'étant actuellement à l'état de développement et n'offrant pas encore les sélectivités atteintes par les membranes polymères, ces matériaux offrent des avantages technologiques certains par rapport aux membranes polymères : meilleure tenue en température, durée de vie plus importante.

A titre d'exemples non exhaustifs, la couche sélective de ces membranes peut être constituée des matériaux suivants :
- alumine (Van Gestel T., Vandecasteele C., Buekenhoudt A., Dotremont C., Luyten J., Van der Bruggen B., Maesc G., "Corrosion Properties of Alumina and Titania NF Membranes", Journal of Membrane Science 214 (2003) 21-29),
- oxyde de titane (I. Voigt, G. Fischer, P. Puhlfürfß, M. Schleifenheimer, M. Stahn, "TiO₂-NF-Membranes on Capillary Supports", Separation and Purification Technology 32 (2003) 87-91 ; S. Benfer, U. Popp, H. Richter, C. Siewert, G. Tomandl, "Development and Characterization of Ceramic Nanofiltration Membranes", Separation and Purification Technology 22-23 (2001) 231-237),
- silice amorphe, silice mésoporeuse (type MCM), silice + zircone (T. Tsuru, M. Miyawaki, H. Kondo, T. Yoshioka, M. Asaeda, "Inorganic Porous Membranes for Nanofiltration of Non-aqueous Solutions", Separation and Purification Technology 32 (2003) 105-109),
- zircone (S. Benfer, U. Popp, H. Richter, C. Siewert, G. Tomandl, "Development and Characterization of Ceramic Nanofiltration Membranes", Separation and Purification Technology 22-23 (2001) 231-237),
- alumine + oxyde de titane (Van Gestel T., Vandecasteele C., Buekenhoudt A., Dotremont C., Luyten J., Van der Bruggen B., Maesc G., "Surface Modification of Alpha-Al₂O₃/TiO₂ Multilayer Membranes for Applications in Non-polar Organic Solvents", Journal of Membrane Science 224 (2003) 3-10).

Il est à noter que, toutes ces phases étant de nature polaire, le transfert de l'alcool sera favorisé par rapport aux composés plus apolaires (tels que les esters ou les triglycérides) du fait d'une meilleure mouillabilité de l'alcool dans la porosité de la couche sélective.

Dans le cas d'une membrane inorganique, il est recommandé de réaliser la séparation membranaire à une température généralement supérieure à la température ambiante et inférieure à 200°C, de préférence inférieure à 150°C, de manière plus préférée à une température allant de 40 à 65°C.

Selon un mode de réalisation de l'invention, l'effluent riche en alcool issu de l'étape de séparation membranaire peut être recyclé à l'entrée de l'étape réactionnelle. Ce recyclage peut avantageusement comprendre le stockage temporaire de l'alcool dans un ballon-tampon.

De préférence, l'effluent riche en alcool issu de l'étape de séparation membranaire peut, avant d'être recyclé à l'entrée de l'étape réactionnelle, subir une étape de purification. Cette étape de purification peut comprendre une ou plusieurs opérations de séparation parmi lesquelles on peut citer une déshydratation de l'alcool, éventuellement une séparation du glycérol de l'alcool ou toute autre séparation connue de l'homme du métier. Cette séparation présente l'avantage d'extraire tout composé polluant de l'alcool qui pourrait inhiber ou ralentir la réaction de transestérification ou en diminuer le rendement.

Selon un autre mode de réalisation, l'effluent riche en esters alkyliques peut être envoyé dans une étape de séparation de l'alcool, par exemple, par vaporisation sous vide, ce qui permet d'extraire les traces d'alcool. L'alcool ainsi séparé est avantageusement recyclé à l'entrée de l'étape réactionnelle. Dans un premier cas, l'alcool issu de cette étape de séparation peut être recyclé directement à l'entrée de l'étape réactionnelle, éventuellement via un ballon-tampon. Dans un deuxième cas, l'alcool issu de cette étape de séparation peut également, avant d'être recyclé à l'entrée de l'étape réactionnelle, subir une étape de purification pouvant comprendre une ou plusieurs opérations de séparation parmi lesquelles on peut citer une déshydratation de l'alcool, éventuellement une séparation du glycérol de l'alcool ou toute autre séparation connue de l'homme du métier. Cette dernière purification a l'avantage d'extraire tout composé polluant de l'alcool qui pourrait inhiber ou ralentir la réaction de transestérification ou en diminuer le rendement.

Selon un mode du procédé de l'invention, les calories de l'effluent issu de l'étape réactionnelle sont utilisées pour réchauffer la charge de ladite étape réactionnelle.

L'étape de séparation est préférentiellement réalisée de manière à ce que l'effluent riche en esters alkyliques présente une teneur en alcool comprise entre 0,1 et 25 % en poids.

Selon un autre mode du procédé de l'invention, on met en oeuvre au moins une première et une deuxième étapes réactionnelles, chacune de ces étapes réactionnelles étant suivie par au moins une étape de séparation membranaire. De préférence, la conversion de l'huile dans la première étape réactionnelle est d'au moins 90 % en poids. La deuxième étape réactionnelle est préférentiellement mise en oeuvre de manière à ce que la teneur en mono-glycérides dans l'effluent issu de ladite étape soit inférieure ou égale à 0,8 % en poids.

### Description détaillée des figures

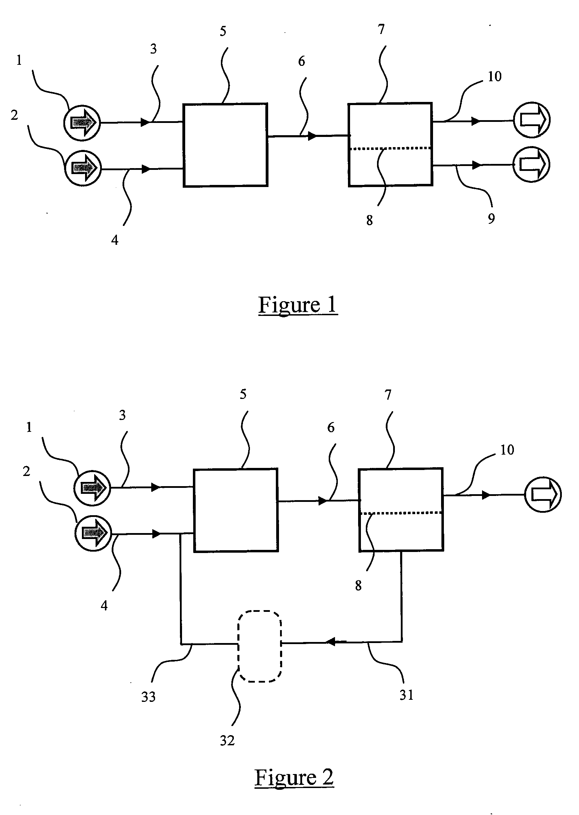

Pour une meilleure compréhension, plusieurs modes de réalisation du dispositif de l'invention sont illustrés par les Figures 1 à 7. Ces modes de réalisation sont donnés à titre d'exemples et ne présentent aucun caractère limitatif. Ces illustrations du dispositif de l'invention ne comportent pas l'ensemble des composantes nécessaires à sa mise en oeuvre. Seuls les éléments nécessaires à la compréhension de l'invention y sont représentés, l'homme du métier étant capable de compléter ces représentations pour réaliser et mettre en oeuvre l'invention.

La Figure 1 représente un mode de réalisation du procédé de l'invention dans lequel des triglycérides 1 et de l'alcool 2 sont respectivement introduits par l'intermédiaire de conduites 3 et 4 dans un réacteur de transestérification 5. Les produits de la réaction sont évacués par une conduite 6 à une pression supérieure à la pression de bulle de ces produits et à une température inférieure à 250°C.

Les produits sont ensuite introduits dans un module de séparation membranaire 7 comportant une membrane 8 qui est sélectivement perméable à l'alcool. Cette opération de séparation membranaire peut, dans ce cas, mettre en oeuvre une membrane de nanofiltration, d'osmose inverse ou une association des deux technologies.

L'alcool est récupéré dans une conduite 9 et constitue l'essentiel du perméat. Le retentat, c'est-à-dire l'ensemble des composés n'ayant pas traversé la membrane, comporte principalement des esters. Ce retentat est récupéré par une conduite 10.

La Figure 2 représente un mode de réalisation du procédé de l'invention comportant la plupart des éléments de la Figure 1 avec, en plus, un recyclage de l'alcool. Dans ce cas, l'alcool récupéré après séparation membranaire est recyclé, par l'intermédiaire d'une conduite 31, dans un ballon-tampon 32, avant d'être envoyé, par une conduite 33, dans le réacteur.

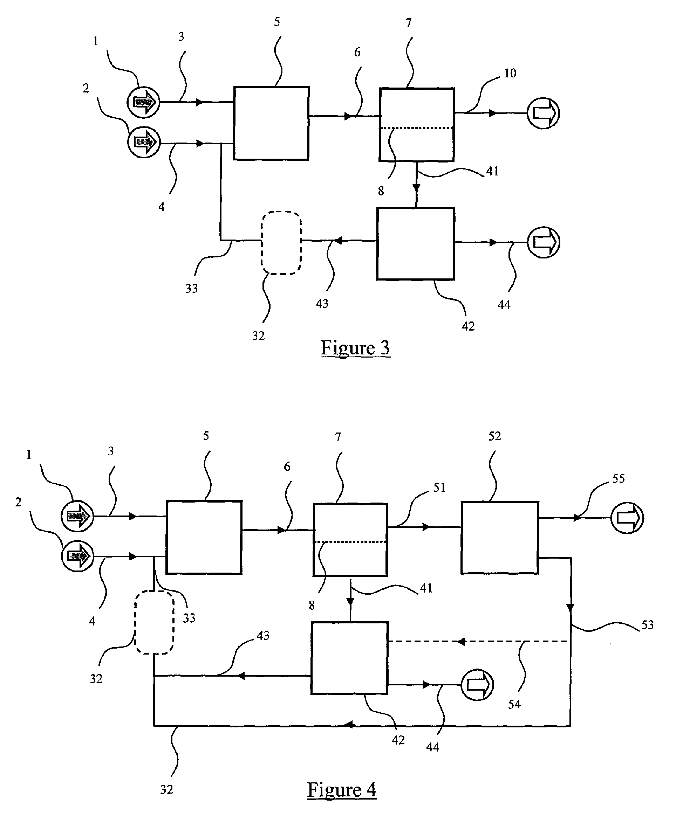

La Figure 3 représente un mode de réalisation du procédé de l'invention comportant la plupart des éléments de la Figure 2 avec, en plus, une étape supplémentaire de purification de l'alcool. Dans ce cas, l'alcool est évacué de l'étape de séparation membranaire par une conduite 41 pour être envoyé dans une étape de séparation 42. L'alcool ainsi purifié est envoyé, par une conduite 43, dans le ballon-tampon 32 avant d'être envoyé, par une conduite 33, dans le réacteur. Les polluants sont, quant à eux, évacués par une conduite 44.

La Figure 4 représente un mode de réalisation du procédé de l'invention comportant la plupart des éléments de la Figure 3 avec, en plus, une étape de séparation de l'alcool issu du retentat. Dans ce cas, le retentat, qui comporte principalement des esters mais également de l'alcool, est envoyé, par une conduite 51, dans une étape de séparation de l'alcool 52, par exemple par vaporisation sous vide. Le flux riche en alcool issu de cette étape de séparation 52 peut être recyclée, par une conduite 53, vers le réacteur via le ballon-tampon 32 ou via l'étape de séparation 42 par l'intermédiaire d'une conduite 54. Le flux riche en esters est, quant à lui, évacué par une conduite 55.

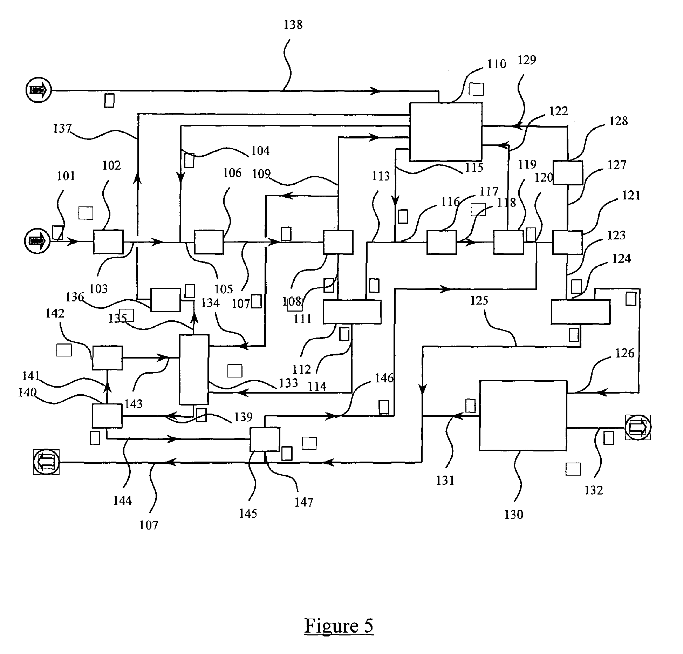

La Figure 5 représente un enchaînement particulier pour la mise en oeuvre du procédé de l'invention.

L'huile à traiter provient généralement d'une unité de trituration de graines de colza ou d'autres huiles végétales comme l'huile de palme, de tournesol, de soja, de copra, de ricin, ainsi que des huiles d'origine animales comme le suif.

On fait passer l'huile de colza brute, via une conduite 101, dans un sécheur sous vide 102 de façon à obtenir une teneur en eau inférieure à 700 ppm en masse. L'huile séchée est envoyée, par l'intermédiaire d'une conduite 103, pour être mélangée avec du méthanol de recycle alimenté par une conduite 104. Le mélange obtenu contient entre 20 % et 80 % en poids, de préférence entre 45 % et 55 % en poids d'huile.

Ce mélange est envoyé, par une conduite 105, successivement dans une pompe (non représenté) pour être comprimé à 6,2 MPa, dans un échangeur (non représenté) pour être chauffé à 473 K, ou 483 K en fin de vie du catalyseur, et dans un réacteur 106. Le réacteur contient un lit fixe d'un catalyseur à base d'aluminate de zinc sous forme d'extrudés. La vitesse volumique horaire au sein du réacteur 106, c'est-à-dire le rapport entre le débit volumique horaire d'huile à traiter sur le volume de catalyseur peut aller de 0,1 à 1,2 h⁻¹, préférentiellement de 0,4 à 0,6 h⁻¹. La conversion en huile obtenue dans ces conditions est d'au moins 90 % masse, généralement d'au moins 92 % en masse.

En sortie du réacteur 106, l'effluent est évacué par une conduite 107. Cet effluent comporte de l'ester, du glycérol, du méthanol et les glycérides peu ou pas convertis (huile, di- et monoglycérides). Cet effluent est ensuite refroidi par échange thermique avec un fluide du procédé devant être chauffé, ce qui permet une bonne intégration thermique du procédé. Classiquement, on met en oeuvre un échangeur "charge-effluent" (non représenté) autour du réacteur. Pour réaliser cet échange thermique, on préchauffe la charge du réacteur, avec son effluent en économisant ainsi une partie de l'énergie de chauffage. La charge du réacteur nécessite souvent un chauffage supplémentaire pour atteindre la température de réaction. L'effluent dans la conduite 107 est ensuite refroidi jusqu'à une température allant de 293 à 423 K, de préférence de 313 à 353 K.

L'effluent est ensuite envoyé, par la conduite 107, dans une série de modules membranaires de nanofiltration 108, maintenus à une pression voisine de 6 MPa. Cette étape de nanofiltration peut être dimensionnée de telle sorte que la teneur en méthanol résiduel dans le mélange soit comprise entre 5 et 25 % en poids, de préférence entre 10 et 20 % en poids. Le méthanol en phase liquide est récupéré dans le perméat, par l'intermédiaire d'une conduite 109, puis est recyclé vers un ballon-tampon 110. Le retentat est, quant à lui, refroidi jusqu'à 323 K et envoyé, par une conduite 111, dans un ballon-décanteur 112 afin de séparer une phase supérieure, riche en ester, qui est évacuée par une conduite 113, d'une phase inférieure, riche en glycérol, qui évacuée par une conduite 114.

La phase riche en ester évacuée du ballon de décantation 112 par la conduite 113 est mélangé avec du méthanol prélevé du ballon-tampon 110 par la conduite 115 de façon à obtenir à nouveau un mélange dont la teneur masse en ester est comprise entre 20 % et 80 % en poids, de préférence entre 45 % et 55 % en poids. On fait passer ce mélange par une conduite 116 de bas en haut dans un second réacteur 117 identique au premier, et opérant dans des conditions opératoires très sensiblement voisines de celles du réacteur 106. Les conditions opératoires de ces réacteurs 106 et 117 sont généralement pratiquement identiques, et le catalyseur utilisé dans chacun des réacteurs est généralement le même. La conversion permet généralement de satisfaire la spécification carburant en mono-glycérides dans l'ester, récupéré dans une conduite 118, qui est généralement au maximum de 0,8 % en poids.

De même que pour la première étape réactionnelle, l'effluent récupéré dans la conduite 118 est refroidi dans un échangeur charge-effluent (non représenté) autour du réacteur 117. Ainsi, la charge du réacteur 117 peut être préchauffée, un chauffage supplémentaire étant généralement nécessaire pour atteindre la température de la réaction.

L'effluent est ensuite envoyé, par la conduite 118, dans une série d'étapes de séparation 119 comportant une étape de nanofiltration, puis par une conduite 120, vers une étape de séparation par évaporation 121, afin d'extraire le méthanol contenu dans l'effluent issu du réacteur 117. L'étape de nanofiltration est réalisée de manière de délivrer un retentat, c'est-à-dire un flux retenu en amont de la membrane, dont la composition est similaire à celle obtenue dans l'étape 108, et la seconde étape de séparation par évaporation 121 est réalisée sous vide de façon à obtenir une phase liquide présentant un maximum de 500 ppm masse de méthanol, de préférence 200 ppm masse. Cela permet le séchage de l'ester à 200 ppm masse d'eau maximum.

Le méthanol issu du perméat de l'étape de nanofiltration 119 est recyclé vers le ballon-tampon 110 par une conduite 122. L'effluent liquide issu de l'étape de séparation par évaporation 121 est envoyé, après refroidissement, par l'intermédiaire d'une conduite 123, dans un décanteur 124, dans lequel on obtient une phase glycérine très pure, envoyée en limite d'unité par une conduite d'évacuation 125, et une phase ester, récupérée par une conduite 126 et qui subit un traitement ultérieur qui sera décrit plus loin. Le méthanol vapeur issu de l'évaporateur 121 est envoyé, par une conduite 127, dans un condenseur 128, puis recyclé, par une conduite 129, vers le ballon-tampon 110.

La phase ester issue du décanteur 124 est ensuite traitée de façon à répondre à la spécification carburant concernant la teneur en glycérol total (libre et potentiel), qui est au maximum de 0,25 % en poids. Ce traitement de l'ester brut peut se faire de différentes façons. Dans le cas présent, l'ester est envoyé par la conduite 126 dans un coalesceur 130, dans lequel les traces de glycérol libre sont éliminées. L'ester peut éventuellement être ensuite envoyé sur les masses adsorbantes d'un adsorbeur, non-représenté, généralement une résine échangeuse d'ions, qui fixe le glycérol dissous. Le glycérol, très pur, séparé de l'ester est envoyé par une conduite 131 en limite d'unité. L'ester final est, quant à lui, récupéré par une conduite 132. Le traitement de l'ester pourrait se faire, dans d'autres cas, grâce à une ou plusieurs étapes de lavage à l'eau de l'ester.

Le flux de glycérine provenant du ballon-décanteur 112, par la conduite 114, est généralement traité de façon à atteindre une teneur maximale en méthanol de 5000 ppm en masse, et une teneur maximale en matières organiques non-glycérineuses, en abrégé MONG, de 1 % en poids, ce qui correspond au niveau commercial généralement acceptable. L'élimination du méthanol contenu dans le flux de glycérine se fait généralement en deux étapes. La première étape est généralement réalisée en fond d'une colonne de distillation 133, ce qui permet d'obtenir une glycérine en fond de colonne ayant une teneur en méthanol jusqu'à 5 % en poids et de séparer l'eau du méthanol sur les plateaux de tête, le méthanol de tête comportant au maximum 800 ppm masse d'eau, de préférence 500 ppm. La colonne 133 est également alimentée par un flux de méthanol venant de l'étape 108 par une conduite 134. Le méthanol purifié sortant en tête de la colonne 133 est envoyé, par une conduite 135, dans un condenseur 136, avant d'être envoyé, par une conduite 137, dans le ballon-tampon 110.

Cette opération est nécessaire pour déconcentrer l'eau qui entre dans l'unité par l'huile de charge 101, dont le séchage est limité à 500 ppm masse. S'il fallait pousser plus loin cette étape de séchage, il faudrait soit augmenter le niveau de vide, ce qui est coûteux, soit augmenter la température, risquant ainsi de décomposer une partie de l'huile. L'autre entrée d'eau peut également provenir du méthanol frais envoyé dans le ballon-tampon 110 par une conduite 138. En utilisant le méthanol commercial le plus sec, c'est-à-dire de grade A, on peut assurer une teneur en eau inférieure à 1000 ppm masse. L'eau entrant dans le système par ces deux voies s'accumule dans la boucle méthanol. L'eau est un inhibiteur du catalyseur, et au-delà d'une teneur en eau de 1000 ppm masse dans le mélange réactionnel, la conversion de l'huile chute sensiblement.

La glycérine extraite du fond de la colonne 133, comportant environ 5 % de méthanol, est envoyée, par une conduite 139, dans un évaporateur sous vide 140. Le méthanol vapeur est envoyé, par une conduite 141, dans un condenseur 142 et recyclé, par une conduite 143, vers la colonne 133. Le flux de glycérine extrait de l'évaporateur 140, contenant environ 3000 ppm de méthanol, est envoyé, par une conduite 144, dans un ballon de décantation 145. La phase ester issue de la tête du ballon-décanteur 145 est renvoyée, par une conduite 146, à l'entrée de l'évaporateur 121 et le glycérol purifié part en limite d'unité, par la conduite 147.

La réaction de transestérification consommant une partie du méthanol, il est nécessaire d'introduire du méthanol frais, par la conduite 138. Une partie de ce méthanol frais est envoyée dans le bac de charge de méthanol, ou ballon-tampon 110, l'autre partie pouvant servir à la régénération des résines échangeuses d'ions utilisées au niveau du traitement de l'ester (non représentées).

### Exemple 1 non conforme à l'invention)

Dans cet exemple, l'extraction de l'alcool en excès, à la sortie des réacteurs de transestérification, est réalisée par évaporation. Les pressions sont données en valeurs absolues.

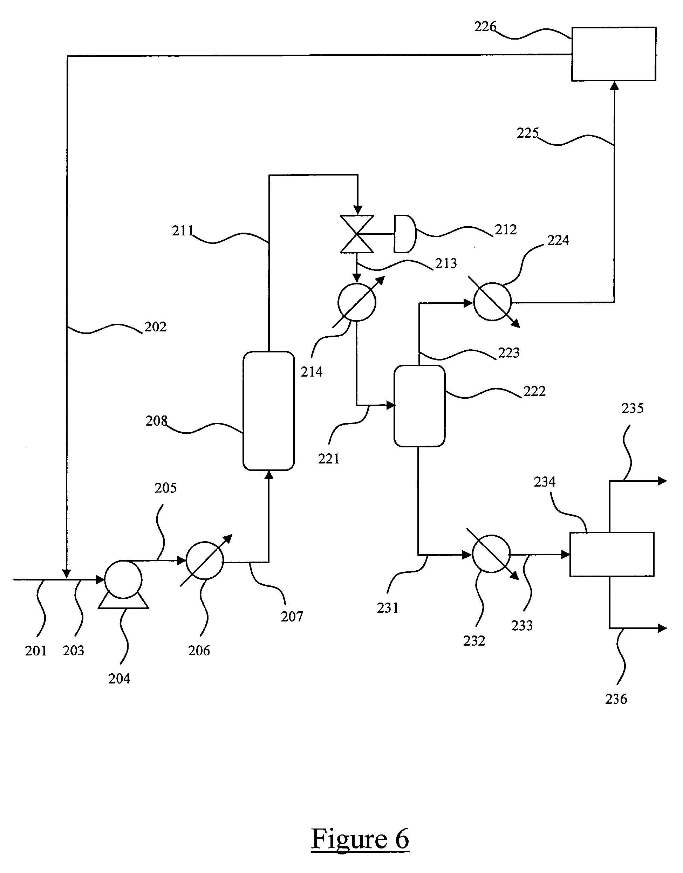

Cet exemple est basé sur un enchaînement selon l'art antérieur représenté à la Figure 6, c'est-à-dire non conforme à la présente invention.

L'huile à traiter provient d'une unité de trituration de graines de colza. On pourrait aussi bien traiter d'autres huiles végétales, comme l'huile de palme, de tournesol, de soja, de copra, de ricin, ainsi que des huiles d'origine animales comme le suif. L'huile de colza brute a été préalablement passée dans un sécheur sous vide de façon à obtenir une teneur en eau inférieure à 700 ppm en masse. On appelle, dans la suite du texte, « huile séchée » l'huile de charge ayant subi ce traitement.

Dans l'enchaînement de la Figure 6, l'huile séchée est alimentée par la conduite 201 pour être ensuite mélangée avec du méthanol de recycle alimenté par une conduite 202, à une pression de 0,25 MPa. Le mélange comportant l'huile séchée et le méthanol de recycle ainsi obtenu comporte 50 % en poids d'huile et présente une température de 323 K. Ce mélange est envoyé, par une conduite 203, dans une pompe 204 pour y être comprimé à 6,2 MPa. Le mélange ainsi comprimé est ensuite envoyé, par l'intermédiaire d'une conduite 205, dans un échangeur de chaleur 206 afin d'y être chauffé à 473 K. Cette température peut atteindre 483 K en fin de vie du catalyseur. Le fluide chaud de cet échange thermique est de la vapeur à haute pression, c'est-à-dire de la vapeur saturante à 2,8 MPa, soit une température de 503 K. Le débit de vapeur nécessaire dans l'échangeur 206, pour cette étape de chauffage des réactifs, est de 1000 kg/h.

Le mélange ainsi chauffé est ensuite envoyé par une conduite 207 de bas en haut dans un réacteur 208 contenant un lit fixe d'un catalyseur à base d'aluminate de zinc sous forme d'extrudés. La vitesse volumique horaire, VVH, c'est-à-dire le rapport entre le débit volumique horaire de charge (huile + méthanol) à traiter sur le volume de catalyseur, est de 0,5 h⁻¹. En sortie du réacteur 208, l'effluent comporte de l'ester, du glycérol, du méthanol et les glycérides peu ou pas convertis (huile, di- et monoglycérides).

Cet effluent est ensuite envoyé, par l'intermédiaire d'une conduite 211, à travers une vanne de détente 212, pour être détendu à une pression voisine de 0,25 MPa. Cette détente a pour effet de vaporiser une grande partie du méthanol contenu dans le mélange. L'effluent ainsi détendu est ensuite envoyé, par une conduite 213, dans un vaporisateur 214 qui a pour fonction d'évaporer une partie de méthanol resté la portion liquide de l'effluent. Cette étape de chauffe se fait par échange thermique avec un débit de 77 kg par heure de vapeur basse pression, c'est-à-dire de la vapeur saturante à 0,50 MPa, soit une température de 425 K. Cette étape d'évaporation est réalisée de telle façon que la teneur en méthanol résiduel non évaporé dans le mélange soit de 15 % en poids.

L'effluent est envoyé, par l'intermédiaire d'une conduite 221, dans un ballon-séparateur de flash 222, dans lequel le méthanol vapeur est séparé du liquide . Le méthanol vapeur est envoyé, par une conduite 223, puis refroidi et condensé à 323 K dans un condenseur 224, avant d'être envoyé, par une conduite 225, vers un ballon-tampon 226. Cette étape de condensation et de refroidissement du liquide se fait par échange thermique avec un débit de 42526 kg/h d'eau de refroidissement arrivant à 30°C et sortant à 40°C. Le méthanol de recycle, qui est mélangé à la charge d'huile, est recyclé du ballon-tampon 226 par la conduite 202.

Le liquide issu du ballon de flash 222 est envoyé, par l'intermédiaire d'une conduite 231, dans un échangeur 232 pour y être refroidi jusqu'à 323 K. Cette étape de refroidissement du liquide se fait par échange thermique avec un débit de 7028 kg/h d'eau de refroidissement arrivant à 30°C et sortant à 40°C. Le liquide est ensuite envoyé, par l'intermédiaire d'une conduite 233, dans un ballon-décanteur 234 pour séparer une phase supérieure riche en ester alimentant la seconde section réactionnelle (non représentée), par l'intermédiaire d'une conduite 235, et une phase inférieure riche en glycérine évacuée par une conduite 236.

### Exemple 2 (selon l'invention)

Dans cet exemple, l'extraction de l'alcool en excès à la sortie des réacteurs de transestérification est réalisée par voie membranaire. Les pressions sont données en valeurs absolues.

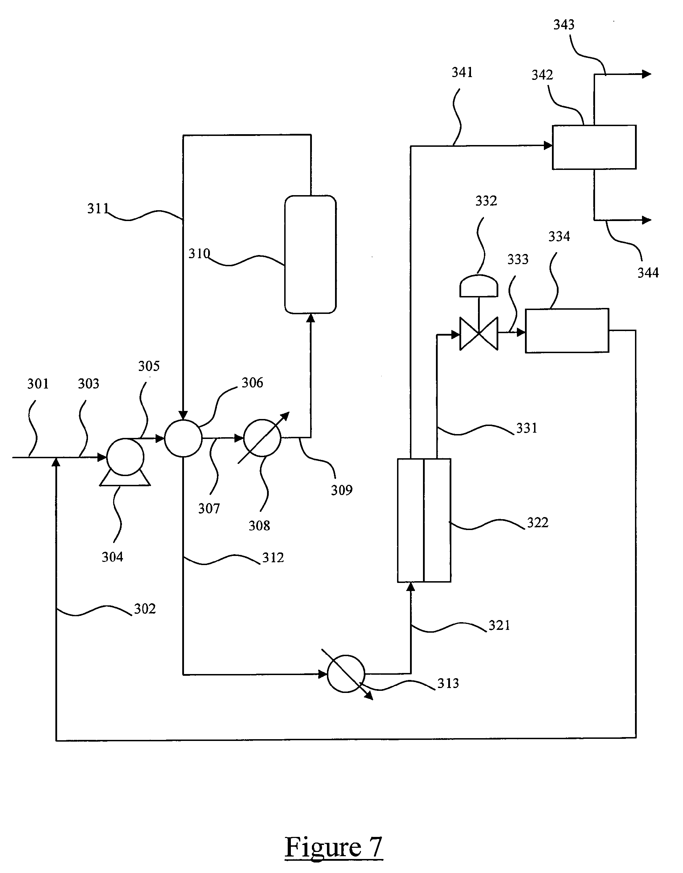

Cet exemple est basé sur un enchaînement selon l'invention représenté à la Figure 7, c'est-à-dire conforme à la présente invention.

Comme dans le cas précédent, l'huile à traiter provient d'une unité de trituration. De la même façon, l'huile de colza brute a subi un séchage sous vide de façon à obtenir une teneur en eau inférieure à 700 ppm en masse.

Dans l'enchaînement de la Figure 7, l'huile séchée est alimentée par la conduite 301 pour être ensuite mélangée avec du méthanol de recycle alimenté par une conduite 302, à une pression de 0,25 MPa. Le mélange d'huile séchée et de méthanol de recycle comporte 50 % en poids d'huile et présente une température de 323 K. Ce mélange est envoyé, par une conduite 303, dans une pompe 304 pour y être comprimé à 0,62 MPa. Le mélange ainsi comprimé est ensuite envoyé, par l'intermédiaire d'une conduite 305, dans un échangeur de chaleur 306 afin d'y être chauffé à 473 K.

L'échangeur 306 est un échangeur du type charge-effluent qui utilise les calories de l'effluent des réacteurs de transestérification pour réchauffer la charge. Ainsi, la charge du réacteur est préchauffée avec son effluent, ce qui permet d'économiser une partie de l'énergie de chauffage et de refroidissement.

Le mélange est envoyé, par une conduite 307, dans un échangeur de chaleur 308 pour être chauffée à 473 K (483 K en fin de vie du catalyseur). Le fluide chaud de cet échange thermique est de la vapeur à haute pression, c'est-à-dire de la vapeur saturante à 2,8 MPa, soit une température de 503 K. Le débit de vapeur nécessaire dans l'échangeur 308, pour cette étape de chauffage des réactifs, est de 492 kg/h.

Le mélange ainsi chauffé est ensuite envoyé, par une conduite 309, de bas en haut dans un réacteur tubulaire 310 contenant un lit fixe d'un catalyseur à base d'aluminate de zinc sous forme d'extrudés. La vitesse volumique horaire, VVH, c'est-à-dire le rapport entre le débit volumique horaire de charge à traiter sur le volume de catalyseur, est de 0,5 h⁻¹. En sortie du réacteur 310, l'effluent comporte de l'ester, du glycérol, du méthanol et les glycérides peu ou pas convertis (huile, di- et monoglycérides).

Cet effluent est ensuite envoyé, par une conduite 311, dans l'échangeur charge-effluent 306 pour être refroidi une première fois. L'effluent ainsi refroidi est envoyé, par une conduite 312, dans un refroidisseur 313, dans lequel il est refroidi une deuxième fois jusqu'à une température de 323 K. Cette étape de refroidissement du liquide se fait par échange thermique avec un débit de 23706 kg/h d'eau de refroidissement arrivant à 30°C et sortant à 40°C.

L'effluent ainsi refroidi est amené, par l'intermédiaire d'une conduite 321, dans une série de modules membranaires de nanofiltration 322, maintenus à une pression voisine de 6 MPa (ou 6 MPa en amont et 1 MPa en aval). Cette étape de nanofiltration est dimensionnée de telle sorte que la teneur en méthanol résiduel dans l'effluent soit de 15 % en poids. Le modèle de membrane utilisé est le STARMEM 120 de MET (Grace Davison). Les modules membranaires sont maintenus à une température de 323 K. Le débit de charge en entrée des modules membranaires est égal à 100 kg/h par m² de membrane installé.

Le méthanol récupéré dans le perméat en phase liquide est envoyé, par une conduite 331, à travers une vanne de régulation de pression 332, avant d'être envoyé par une conduite 333, vers un ballon-tampon 334. Le méthanol de recycle, qui est mélangé à la charge d'huile, est recyclé du ballon-tampon 334 par la conduite 302.

Le liquide récupéré dans l'autre partie des modules membranaires de nanofiltration 322 est envoyé, par l'intermédiaire d'une conduite 341, dans un ballon-décanteur 342 pour séparer une phase supérieure riche en ester alimentant la seconde section réactionnelle (non représentée), par l'intermédiaire d'une conduite 343, et une phase inférieure riche en glycérine évacuée par une conduite 344.

Le Tableau 2 ci-dessous a pour objectif de récapituler la consommation d'eau et de vapeur d'eau dans les cas des Exemples 1 et 2.

**Tableau 2**

| | Exemple 1 | Exemple 2 |
|---|---|---|
| Vapeur haute pression (kg/h) | 1000 | 493 |
| Vapeur basse pression (kg/h) | 77 | 0 |
| Eau de refroidissement (kg/h) | 49554 | 23706 |

Dans les schémas des Exemples 1 et 2, les performances en termes de capacité d'huile traitée, de conversion et de qualité d'ester produit sont les mêmes. Les résultats du Tableau 2 montrent que l'utilisation de modules membranaires conduit à une réduction de 50,7 % de la consommation de vapeur haute pression, à une réduction de 52 % de la consommation de l'eau de refroidissement, et à une suppression totale de la consommation de la vapeur basse pression.

## Revendications

1. Procédé de fabrication d'esters alkyliques d'acide gras et de glycérol comprenant :
- au moins une étape réactionnelle dans laquelle on met en contact une charge comportant une huile végétale et/ou animale et un alcool, en présence d'un catalyseur hétérogène, de manière à obtenir un effluent comportant au moins des esters alkyliques, du glycérol et de l'alcool,
- et au moins une étape de séparation lors de laquelle on réalise une séparation d'au moins une portion de l'effluent de manière à séparer un effluent riche en alcool et un effluent riche en esters alkyliques,
ledit procédé étant **caractérisé en ce qu'**au moins une étape de séparation comporte une séparation membranaire à l'aide d'au moins une membrane perméable à l'alcool.

2. Procédé selon la revendication 1, **caractérisé en ce que** l'alcool est essentiellement constitué par du méthanol et/ou de l'éthanol.

3. Procédé selon l'une quelconque des revendications 1 et 2, **caractérisé en ce qu'**au moins une séparation membranaire est réalisée par nano-filtration.

4. Procédé selon l'une quelconque des revendications 1 et 2, **caractérisé en ce qu'**au moins une séparation membranaire est réalisée par osmose inverse.

5. Procédé selon l'une quelconque des revendications 1 à 4, **caractérisé en ce que** la (ou les) membrane(s) utilisée(s) dans au moins une étape de séparation est (sont) au moins partiellement perméable(s) au glycérol.

6. Procédé selon l'une quelconque des revendications 1 à 5, **caractérisé en ce qu'**au moins une membrane comporte un film à base de polymère.

7. Procédé selon l'une quelconque des revendications 1 à 6, **caractérisé en ce qu'**on met en oeuvre au moins une membrane inorganique.

8. Procédé selon l'une quelconque des revendications 1 à 7, **caractérisé en ce que** l'effluent riche en alcool issu de l'étape de séparation membranaire est recyclé à l'entrée de l'étape réactionnelle.

9. Procédé selon la revendication 8, **caractérisé en ce que** le recyclage comprend le stockage temporaire de l'alcool dans un ballon-tampon.

10. Procédé selon l'une quelconque des revendications 8 et 9, **caractérisé en ce que** l'effluent riche en alcool issu de l'étape de séparation membranaire, avant d'être recyclé à l'entrée de l'étape réactionnelle, subit une étape de purification.

11. Procédé selon l'une quelconque des revendications 1 à 10, **caractérisé en ce que** l'effluent riche en esters alkyliques est envoyé dans une étape de séparation de l'alcool.

12. Procédé selon la revendication 11, **caractérisé en ce que** l'alcool issu de l'étape de séparation de l'effluent riche en esters alkyliques est recyclé à l'entrée de l'étape réactionnelle.

13. Procédé selon la revendication 12, **caractérisé en ce que** l'alcool issu de l'étape de séparation de l'effluent riche en esters alkyliques, avant d'être recyclé à l'entrée de l'étape réactionnelle, subit une étape de purification.

14. Procédé selon l'une quelconque des revendications 1 à 13, **caractérisé en ce que** les calories de l'effluent issu de l'étape réactionnelle sont utilisées pour réchauffer la charge de ladite étape réactionnelle.

15. Procédé selon l'une quelconque des revendications 1 à 14, **caractérisé en ce que** l'étape de séparation est réalisée de manière à ce que l'effluent riche en esters alkyliques présente une teneur en alcool comprise entre 0,1 et 25 % en poids.

16. Procédé selon l'une quelconque des revendications 1 à 15, **caractérisé en ce qu'**on met en oeuvre au moins une première et une deuxième étapes réactionnelles, chacune de ces étapes réactionnelles étant suivie par au moins une étape de séparation membranaire.

17. Procédé selon la revendication 16, **caractérisé en ce que** la conversion de l'huile dans la première étape réactionnelle est d'au moins 90 % en poids.

18. Procédé selon l'une quelconque des revendications 16 et 17, **caractérisé en ce que** la deuxième étape réactionnelle est mise en oeuvre de manière à ce que la teneur en mono-glycérides dans l'effluent issu de ladite étape est inférieure ou égale à 0,8 % en poids.
